# EUROPÄISCHE PATENTSCHRIFT

(11) **EP 4 048 336 B1**
(45) Veröffentlichungstag und Bekanntmachung des Hinweises auf die Patenterteilung: **29.05.2024**
(21) Anmeldenummer: 20800567.8
(22) Anmeldetag: 23.10.2020
(51) Int. Cl.: A61M 1/14

(54) **DIALYSESYSTEM MIT KONTINUIERLICHER GLUKOSE-ÜBERWACHUNG**
DIALYSIS SYSTEM WITH CONTINUOUS GLUCOSE MONITORING
SYSTÈME DE DIALYSE AVEC SURVEILLANCE DE LA GLYCÉMIE EN CONTINU

(30) Priorität: 25.10.2019 DE 102019128847
(43) Veröffentlichungstag der Anmeldung: 31.08.2022
(73) Patentinhaber: Fresenius Medical Care Deutschland GmbH, 61352 Bad Homburg (DE)
(72) Erfinder: WABEL, Peter, 61191 Rosbach (DE)
(74) Vertreter: Herrmann, Uwe
(86) Internationale Anmeldenummer: PCT/EP2020/079958
(87) Internationale Veröffentlichungsnummer: WO 2021/078975

(56) Entgegenhaltungen:
- EP-A1- 2 859 911
- US-A1- 2012 296 253
- US-A1- 2014 148 749
- US-A1- 2019 083 011

## Beschreibung

Die Erfindung betrifft ein System zur Durchführung einer Peritonealdialysebehandlung, das eine kontinuierliche Überwachung des Blutzuckerspiegels eines behandelten Patienten (cGM = Continuous Glucose Monitoring) vorsieht.

Das Glukosemolekül hat eine Molekularmasse von etwa 180 g/mol und bindet nicht an Plasmaproteine. Es kann daher während einer Dialysebehandlung in einfacher Weise durch die Membran eines Dialysefilters (Hämodialyse) oder das Peritoneum (Peritonealdialyse) diffundieren, was je nach dem Gradienten zu einer Erhöhung oder Verringerung des Blutzuckerspiegels führen kann.

Bei Patienten mit einer schlechten Nahrungsaufnahme, verringerten Glukoneogenese oder reduzierter Insulinausschüttung ist das Risiko einer dialyseindizierten Hypoglykämie besonders hoch. Eine Studie zeigte, dass am Tag einer Dialysebehandlung bei 9 von 10 Patienten wenigstens ein Blutzuckerwert von kleiner 70 mg/dl und bei 4 von 10 Patienten wenigstens ein Blutzuckerwert von kleiner 55 mg/dl gemessen wird. All diese hypoglykämischen Episoden waren asymptomatisch und wären ohne spezifisches Monitoring nicht zu erkennen. Daraus resultiert eine besondere Gefahr, da Hypoglykämie einen Risikofaktor für kardiovaskuläre Ereignisse wie etwa Schlaganfälle sowie für Augen- und Nervenschäden bei Dialysepatienten darstellt.

US 2012/296253 A1 betrifft die Nachrüstung von Hämodialysegeräten und deren Steuerung sowie deren Verwendung zur Behandlung von proliferativen Erkrankungen. EP 2 859 911 A1 betrifft eine Vorrichtung für einen vaskulären Zugang, die sowohl die Blutüberwachung als auch den Zugang zu einem in einem Hämodialysepatienten implantierten Gefäßelement ermöglicht. US 2014/148749 A1 bezieht sich auf ein Verfahren und eine Vorrichtung zur Steuerung und Einstellung des Glukosespiegels im Blut eines Patienten während der Dialyse. US 2019/083011 A1 betrifft Geräte zur kontinuierlichen Glukosemessung, die einem Menschen implantiert werden können und die Glukosekonzentration in der Peritonealflüssigkeit überwachen können, sowie ein implantierbares Insulinreservoir, dessen Betrieb mit dem implantierbaren kontinuierlichen Glukoseüberwachungssystem gekoppelt werden kann.

Aufgabe der Erfindung ist es, die Häufigkeiten und Intensitäten glykämischer Episoden bei Dialysepatienten zu verringern.

Vor diesem Hintergrund betrifft die Erfindung ein System zur Durchführung einer Dialysebehandlung mit einem Dialysegerät, einer Steuereinheit und einem Sensor, wobei der Sensor mit der Steuereinheit signalverbunden ist, wobei die Steuereinheit ausgebildet ist, anhand des Sensors den Blutzuckerspiegel oder den interstitiellen Glukosewert eines Patienten während einer Dialysebehandlung zu messen, auf der Grundlage der Messwerte einen zeitlichen Verlauf des Blutzuckerspiegels oder der interstitiellen Glukosewerte aufzuzeichnen und unter Berücksichtigung des Verlaufs einen oder mehrere Behandlungsparameter des Dialysegeräts automatisch anzupassen oder eine Handlungsanweisung für eine benutzerseitige Anpassung eines Behandlungsparameters oder ein sonstiges benutzerseitiges Verhalten auszugeben. Es handelt sich bei dem Dialysegerät um ein Peritonealdialysegerät.

Im Rahmen der Erfindung ist also vorgesehen, eine kontinuierliche Überwachung des Blutzuckerspiegels des Patienten (cGM = Continuous Glucose Monitoring) vorzunehmen. Dadurch können etwaige glykämische Episoden des Patienten, also Hypo- oder Hyperglykämien erkannt und in der aktuellen Behandlung oder in künftigen Therapieverschreibungen berücksichtigt werden. Generell erlaubt das cGM Rückschlüsse auf die patientenspezifische Fähigkeit zur Glukoseabsorption, sodass eine besser auf den individuellen Patienten abgestimmte Planung der Therapieverschreibung ermöglicht wird.

Die Anpassung des bzw. der Behandlungsparameter einer aktuellen Dialysebehandlung bzw. die Ausgabe einer entsprechenden Handlungsanweisung erfolgt im Rahmen der Erfindung vorzugsweise auf der Grundlage von Verläufen des Blutzuckerspiegels oder der interstitiellen Glukosewerte, die im Rahmen vergangener Dialysebehandlung bestimmt wurden.

Die Steuereinheit ist vorzugsweise Teil des Dialysegeräts in dem Sinne, dass sie darin verbaut ist.

In einer bevorzugten Ausführungsform kann die Steuereinheit ausgebildet sein, ferner Informationen über die Körperzusammensetzung des Patienten zu beziehen und diese Informationen bei der automatischen Anpassung der Behandlungsparameter oder Ausgabe entsprechender Informationen einfließen zu lassen. Das System kann zur Ermittlung der Informationen über die Körperzusammensetzung eigene Sensoren umfassen. Alternativ kann die Steuereinheit ausgebildet sein, diese Informationen über ein Benutzerinterface oder ein externes Gerät zu beziehen, das anhand einer Schnittstelle mit dem System in Verbindung steht. Informationen über die Körperzusammensetzung umfassen beispielsweise Informationen über den Fettgehalt oder die Muskelmasse des Patienten. Insbesondere kann die Steuereinheit ausgebildet sein, Informationen über den Flüssigkeitsstatus des Patienten zu verwenden. Es kann sich um aktuell bestimmte oder historische, dabei aber vorzugsweise in regelmäßigen Abständen aktualisierte Daten handeln.

Die Anpassung der Behandlungsparameter kann unter anderem eine Anpassung der Glukosekonzentration einer an den Patienten verabreichten Dialyselösung umfassen. Eine automatische Anpassung der Glukosekonzentration kann beispielsweise an Dialysegeräten erfolgen, die eine automatische Zubereitung der Dialyselösung aus Konzentraten vornehmen. Alternative kann der Patient bzw. Benutzer aufgefordert werden, der Dialysemaschine für die Behandlung eine Dialyselösung mit einer bestimmten Konzentration zuzuführen.

Weiterhin kann die Anpassung der Behandlungsparameter eine Anpassung der Behandlungsdurchführung umfassen. Im Falle einer Peritonealdialyse kann beispielsweise die Verweildauer der Dialyselösung im Peritoneum des Patienten während eines Zyklus (die Dwell-Zeit) angepasst werden.

In einer Ausführungsform umfasst das System ferner eine Vorrichtung zur Verabreichung von Wirkstoffen an den Patienten und die Anpassung der Behandlungsparameter umfasst eine Verabreichung von Wirkstoffen an den Patienten. Die Vorrichtung zur Verabreichung von Wirkstoffen an den Patienten kann im Dialysegerät integriert sein oder separat vorliegen. Beispielsweise kann vorgesehen sein, dass das System bzw. das Dialysegerät eine Insulinpumpe aufweist und dass bei Vorliegen einer hyperglykämischen Episode oder der Gefahr einer hyperglykämischen Episode Insulin an den Patienten verabreicht wird. Weiterhin kann die Gabe eines Diuretikums vorgesehen sein, wenn Informationen zu hohen Wassereinlagerungen vorliegen.

In einer weiteren Ausführungsform kann die Steuereinheit ausgebildet sein, Informationen über das Plasmavolumen des Patienten zu beziehen und diese Informationen bei der automatischen Anpassung der Behandlungsparameter oder Ausgabe entsprechender Informationen einfließen zu lassen. Diese Information spielt im Rahmen der Hämodialyse eine Rolle. Zur Ermittlung der entsprechenden Informationen kann das System entweder eigene Sensoren umfassen oder ausgebildet sein, die Informationen über ein Benutzerinterface oder ein externes Gerät zu beziehen.

Ebenfalls kann die Anpassung der Behandlungsparameter eine Anpassung der Ultrafiltrationsrate umfassen. Dies spielt im Rahmen der Hämodialyse eine Rolle.

Bei dem Sensor zur Bestimmung des Blutzuckerspiegels des Patienten kann es sich in einer Ausführungsform um ein Implantat handeln. Die Messung des Blutzuckerspiegels erfolgt also vorzugsweise anhand eines implantierten Sensors, dessen Messwerte drahtlos an der Steuereinheit des Systems abgefragt werden können.

Alternativ oder zusätzlich zu einem implantierten Sensor kann die Blutzuckerbestimmung auch nicht-invasiv über die Haut erfolgen.

Als Sensoren eignen sich u.a. enzymatische oder auch amperometrische Sensoren.

Weiterhin kann eine weitere Handlungsempfehlung an den Patienten die Angabe einer aus den Messwerten abgeleitete Insulindosis beinhalten, die sich der Patient vor Beginn der Behandlung z.B. mittels eines Insulin-Pen appliziert.

In einer Ausführungsform ist vorgesehen, dass das System ferner ein Benutzerinterface oder eine Schnittstelle zur Datenverbindung mit einem externen Gerät aufweist. Das Benutzerinterface ist ebenso wie die Steuereinheit vorzugsweise Teil des Dialysegeräts. Bei dem externen Gerät kann es sich beispielsweise um ein Smartphone oder einen Computer handeln. Die Schnittstelle kann beispielsweise zur drahtlosen Übertragung der Daten ausgebildet sein.

In einer Ausführungsform ist die Steuereinheit ausgebildet, die Handlungsanweisung für eine benutzerseitige Anpassung an einem Benutzerinterface anzuzeigen oder an ein externes Gerät zu übermitteln.

Weitere Einzelheiten und Vorteile der Erfindung ergeben sich aus den nachfolgend anhand der Figuren beschriebenen Ausführungsbeispielen. In den Figuren zeigen:
- Figur 1:: eine schematische Darstellung eines Workflow bei der Anwendung der Erfindung auf eine Peritonealdialysebehandlung; und
- Figur 2:: ein Diagramm zu beispielhaften Verläufen des Blutzuckerspiegels und Blutdrucks während einer Hämodialysebehandlung.

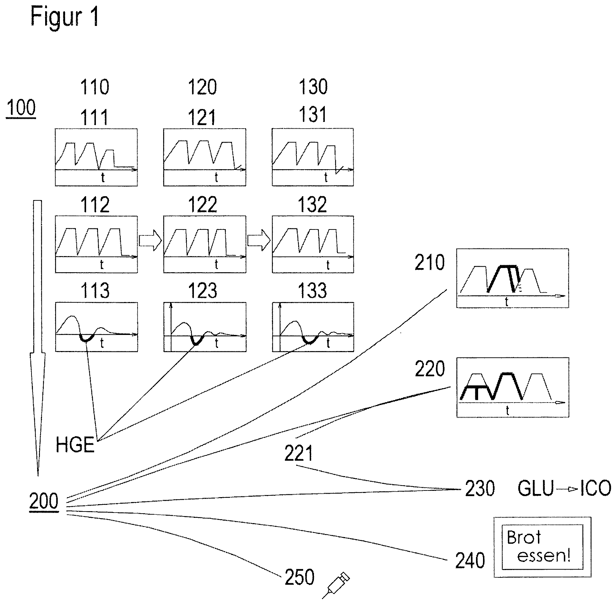

Figur 1 bezieht sich auf eine Anwendung des erfindungsgemäßen Konzepts auf die Peritonealdialyse.

In einem ersten Schritt 100 werden zunächst mehrere Behandlungen 110, 120 und 130 durchgeführt, wobei die Behandlungsverläufe 111, 121 und 131 zu vorgegebenen Verschreibungen 112, 122 und 132 korrespondieren und mehrere Zyklen mit jeweils einer Inflow-Phase, einer Dwell-Phase und einer Outflow-Phase umfassen. Die Zusammensetzung der verabreichten Dialyselösung ist ebenfalls vorgegeben und kann zwischen den Zyklen unterschiedlich sein. In Übereinstimmung mit der Erfindung ist vorgesehen, dass während dieser Behandlungen jeweils die Verläufe 113, 123 und 133 des Blutzuckerspiegels oder der interstitiellen Glukosewerte des Patienten bestimmt werden. Im gezeigten Beispielfall treten in allen drei Behandlungen hypoglykämische Episoden HGE auf.

Wird wie im gezeigten Beispielfall über mehrere Behandlungen 110, 120 und 130 hinweg mittels einer kontinuierlichen Bestimmung des Glukosegehalts im Blut des Patienten festgestellt, dass dieser in regelmäßigen Abständen zu hypoglykämischen Episoden HGE neigt, setzt in einem zweiten Schritt 200 die Steuereinheit des Geräts Maßnahmen, um derartige Episoden HGE zu vermeiden bzw. deren Auftreten und Intensität zumindest einzudämmen. um dem entgegenzuwirken.

Eine Maßnahme 210 kann in der Anpassung der Verschreibung bzw. Behandlung liegen. Beispielsweise kann für einen Zyklus (Inflow-Dwell-Outflow), während dessen auf der Grundlage der Daten eine hypoglykämische Episode zu erwarten ist, die Glukosekonzentration in der Dialyselösung automatisch auf beispielsweise 4,25% Glukose erhöht und/oder die Dwell-Zeit automatisch verlängert werden. Auch kann beispielsweise an einem Benutzerinterface des Geräts ein Signal ausgegeben werden, welches dem Benutzer anzeigt, dass für den fraglichen Zyklus eine Dialyselösung mit einer höheren Glukosekonzentration verwendet oder die Dwell-Zeit verlängert werden soll.

Weiterhin kann das Gerät im Rahmen einer möglichen Maßnahme 220 den Flüssigkeitsstatus 221, also das Ausmaß der Flüssigkeitseinlagerungen des Patienten bei der Anpassung der Behandlung bzw. entsprechenden Signalausgabe berücksichtigen. Beispielsweise kann bei einem Patienten mit einem vergleichsweise niedrigen Körperwassergehalt bzw. Flüssigkeitsstatus, der oft mit einer Hypogläkämie einhergeht, die Therapie derart angepasst werden, dass zwei kurze Zyklen mit einer Dialyselösung mit niedrigem Glukosegehalt und ein nachfolgender Zyklus mit einer Dialyselösung mit höherem Glukosegehalt durchgeführt werden. Die Anpassung kann automatisch erfolgen oder es können entsprechende Anweisungen an den Benutzer ausgegeben werden. Alternativ kann auch die Dwellzeit angepasst werden.

Neigt ein Patient zu regelmäßigen hyperglykämischen Episoden und weist entsprechend einen vergleichsweise hohen Körperwassergehalt bzw. Flüssigkeitsstatus auf, so kann im Rahmen einer weiteren denkbaren Maßnahme 230 vorgesehen sein, auf der Grundlage einer kontinuierlichen Bestimmung des Glukosegehalts im Blut oder des interstitiellen Glukosewerts und unter zusätzlicher Berücksichtigung des Fluidstatus den Glukosegehalt der verabreichten Dialyselösungen so anzupassen, dass hyperglykämische Episoden vermieden werden. Neben einer Verringerung oder Erhöhung des Glukosegehalts der Dialyselösung besteht auch die Möglichkeit eines teilweisen oder vollständigen Ersatzes der Glukose durch Icodextrin oder andere Osmotika.

An Stelle einer Anpassung eines Behandlungsparameters wie etwa eines Glukosegehalts der verabreichten Lösung kann auch eine Handlungsanweisung 240 an den Patienten angegeben werden, die sich auf sein Verhalten vor bzw. während der Therapie bezieht. Beispielsweise kann der Patient aufgefordert werden, vor Beginn der Therapie noch einen Snack zu sich zu nehmen.

Sollten die genannten Maßnahmen nicht ausreichen bzw. als nicht ausreichend prognostiziert werden, um eine hyperglykämische Episode zu vermeiden, kann die Steuereinheit ausgebildet sein, eine Insulingabe 250 mittels einer Insulinpumpe zu initiieren. Alternativ kann die Glukose durch Icodextrin ersetzt werden, wenn eine übermäßige Zunahme des Fettgehalts beobachtet wird.

Basierend auf einer Bestimmung der Körperzusammensetzung des Patienten (BCM - Body Composition Measurement) können zudem der Körperfettgehalt und die Muskelmasse sowie letztlich der Lean Tissue Index (LTI) bzw. die Lean Tissue Mass (LTM) in regelmäßigen Abständen bestimmt werden, um unter anderem den Ernährungszustand des Patienten zu erfassen. Sollte dabei festgestellt werden, dass diese Parameter mit der Zeit abnehmen, kann eine höhere Glukosekonzentration, gegebenenfalls in Kombination mit der Gabe von Insulin, zur Unterstützung des Aufbaus von Körperfett vorgesehen werden.

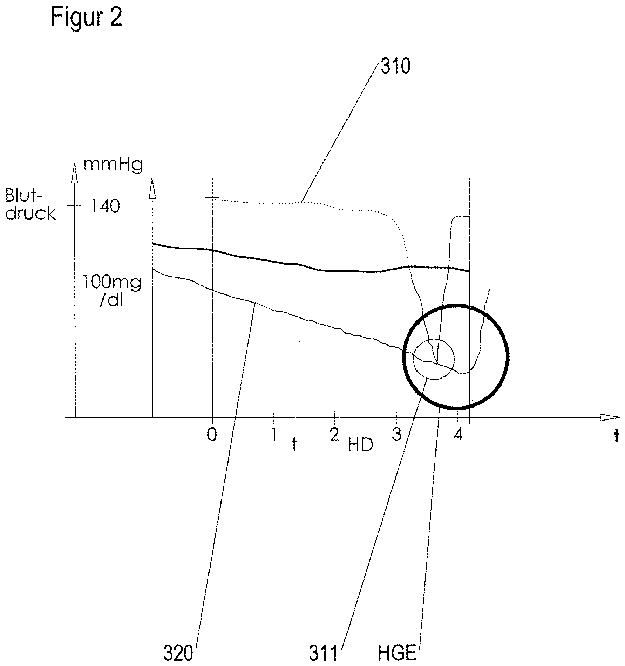

Figur 2 bezieht sich auf eine Anwendung des Konzepts auf die Hämodialyse.

Auch in der Hämodialyse können die Behandlungsparameter angepasst werden, um glykämische Episoden am Patienten zu vermeiden. Neben dem Verlauf des Blutzuckerspiegels bzw. des interstitiellen Glukosewerts sowie des Fluidstatus des Patienten können hier insbesondere noch das Plasmavolumen des Patienten zur Anpassung der Behandlungsparameter herangezogen werden, wobei die Anpassung der Behandlungsparameter hier insbesondere auch eine Anpassung der Ultrafiltrationsrate umfassen kann.

Beispielsweise kann es während einer Hämodialyse zu einem Abfall des Blutdrucks kommen. Die Kurve 310 der Figur 2 zeigt einen beispielhaften Verlauf des Blutdrucks. Ein Abfall 311 des Blutdrucks ist ein typisches Symptom für das Vorliegen einer hypoglykämischen Episode HGE, wobei der Blutdruckabfall natürlich auch andere Ursachen wie etwa eine starke Abnahme des Plasmavolumens haben kann. Die Kurve 320 der Figur 2 zeigt einen beispielhaften Verlauf des Blutzuckerspiegels, der in diesem Fall mit dem Abfall des Blutdrucks einhergeht. Anhand der kontinuierlichen Bestimmung des Blutzuckerspiegels lassen sich aber jedenfalls Rückschlüsse darauf ziehen, ob ein Abfall des Blutzuckerspiegels ursächlich für den Blutdruckabfall sein könnte, oder nicht.

## Patentansprüche

1. System zur Durchführung einer Dialysebehandlung mit einem Dialysegerät, einer Steuereinheit und einem Sensor, wobei der Sensor mit der Steuereinheit signalverbunden ist, wobei die Steuereinheit ausgebildet ist, anhand des Sensors den Blutzuckerspiegel oder den interstitiellen Glukosewert eines Patienten während einer Dialysebehandlung zu messen, auf der Grundlage der Messwerte einen zeitlichen Verlauf des Blutzuckerspiegels oder der interstitiellen Glukosewerte aufzuzeichnen und unter Berücksichtigung des Verlaufs einen oder mehrere Behandlungsparameter des Dialysegeräts automatisch anzupassen oder eine Handlungsanweisung für eine benutzerseitige Anpassung eines Behandlungsparameters oder ein sonstiges benutzerseitiges Verhalten auszugeben, **dadurch gekennzeichnet, dass** es sich bei dem Dialysegerät um ein Peritonealdialysegerät handelt.

2. System nach Anspruch 1, **dadurch gekennzeichnet, dass** die Steuereinheit ausgebildet ist, Informationen über die Körperzusammensetzung des Patienten zu beziehen und diese Informationen bei der automatischen Anpassung der Behandlungsparameter oder Ausgabe entsprechender Informationen einfließen zu lassen.

3. System nach einem der vorhergehenden Ansprüche, **dadurch gekennzeichnet, dass** die Anpassung der Behandlungsparameter eine Anpassung der Glukosekonzentration einer an den Patienten verabreichten Dialyselösung umfasst.

4. System nach einem der vorhergehenden Ansprüche, **dadurch gekennzeichnet, dass** die Anpassung der Behandlungsparameter eine Anpassung der Behandlungsdurchführung umfasst.

5. System nach einem der vorhergehenden Ansprüche, **dadurch gekennzeichnet, dass** das System ferner eine Vorrichtung zur Verabreichung von Wirkstoffen an den Patienten aufweist und dass die Anpassung der Behandlungsparameter eine Verabreichung von Wirkstoffen an den Patienten umfasst.

6. System nach einem der vorhergehenden Ansprüche, **dadurch gekennzeichnet, dass** die Steuereinheit ausgebildet ist, die Handlungsanweisung für eine benutzerseitige Anpassung an einem Benutzerinterface anzuzeigen oder an ein externes Gerät zu übermitteln.

## Claims

1. System for carrying out a dialysis treatment using a dialysis machine, a control unit, and a sensor, wherein the sensor is in signal communication with the control unit, wherein the control unit is configured to measure the blood glucose level or the interstitial glucose value of a patient using the sensor during a dialysis treatment, to record a time progression of the blood glucose level or of the interstitial glucose values on the basis of the measurement values, and to automatically adapt one or more treatment parameters of the dialysis machine while taking into account the progression or to output instructions for a user-side adaptation of a treatment parameter or to output another user-side behavior, **characterized in that** the dialysis machine is a peritoneal dialysis machine.

2. System in accordance with claim 1, **characterized in that** the control unit is configured to obtain information on the body composition of the patient and to have this information enter into the automatic adaptation of the treatment parameters or into the output of corresponding information.

3. System in accordance with one of the preceding claims, **characterized in that** the adaptation of the treatment parameters comprises an adaptation of the glucose concentration of a dialysis solution administered to the patient.

4. System in accordance with one of the preceding claims, **characterized in that** the adaptation of the treatment parameters comprises an adaptation of the treatment performance.

5. System in accordance with one of the preceding claims, **characterized in that** the system furthermore comprises an apparatus for administering active ingredients to the patient; and **in that** the adaptation of the treatment parameters comprises an administration of active ingredients to the patient.

6. System in accordance with one of the preceding claims, **characterized in that** the control unit is configured to display the instructions for a user-side adaptation at a user interface or to transmit them to an external device.

## Revendications

1. Système permettant de mettre en oeuvre un traitement de dialyse comportant un appareil de dialyse, une unité de commande et un capteur, le capteur étant relié par signal à l'unité de commande, l'unité de commande étant conçue pour mesurer, au moyen du capteur, le taux de glycémie ou la valeur du glucose interstitiel d'un patient pendant un traitement de dialyse, enregistrer une variation dans le temps du taux de glycémie ou de la valeur du glucose interstitiel sur la base des valeurs de mesure et adapter automatiquement un ou plusieurs paramètres de traitement de l'appareil de dialyse compte tenu de la variation ou fournir en sortie une instruction pour une adaptation à effectuer par l'utilisateur d'un paramètre de traitement ou une autre action à effectuer par l'utilisateur, **caractérisé en ce que** l'appareil de dialyse est un appareil de dialyse péritonéale.

2. Système selon la revendication 1, **caractérisé en ce que** l'unité de commande est conçue pour recevoir des informations concernant la composition corporelle du patient et prendre en compte ces informations lors de l'adaptation automatique des paramètres de traitement ou de la sortie d'informations correspondantes.

3. Système selon l'une des revendications précédentes, **caractérisé en ce que** l'adaptation des paramètres de traitement comprend une adaptation de la concentration en glucose d'une solution de dialyse administrée au patient.

4. Système selon l'une des revendications précédentes, **caractérisé en ce que** l'adaptation des paramètres de traitement comprend une adaptation de la mise en oeuvre du traitement.

5. Système selon l'une des revendications précédentes, **caractérisé en ce que** le système présente en outre un dispositif permettant d'administrer des substances actives au patient et **en ce que** l'adaptation des paramètres de traitement comprend l'administration de substances actives au patient.

6. Système selon l'une des revendications précédentes, **caractérisé en ce que** l'unité de commande est conçue pour afficher l'instruction pour une adaptation à effectuer par l'utilisateur sur une interface utilisateur ou la transmettre à un appareil externe.
